# FASCICULE DE BREVET EUROPEEN

(11) **EP 2 760 482 B1**
(45) Date de publication et mention de la délivrance du brevet: **04.05.2022**
(21) Numéro de dépôt: 12784339.9
(22) Date de dépôt: 27.09.2012
(51) Int. Cl.: A61L 2/18, C11D 3/395, C11D 3/48, C11D 3/39

(54) **MÉTHODE DE TRAITEMENT DE SURFACES CONTAMINÉES PAR LE PRION**
VERFAHREN ZUR BEHANDLUNG VON DURCH PRIONEN KONTAMINIERTEN OBERFLÄCHEN
METHOD FOR TREATING SURFACES CONTAMINATED BY PRIONS

(30) Priorité: 27.09.2011 FR 1158654
(43) Date de publication de la demande: 06.08.2014
(73) Titulaire: Commissariat à l'Énergie Atomique et aux Énergies Alternatives, 75015 Paris (FR)
(72) Inventeur: DESLYS, Jean-Philippe, 78170 La Celle-Saint-Cloud (FR); COMOY, Emmanuel, 91190 Saint-Aubin (FR); WODLING, Pascal, 92260 Antony (FR)
(74) Mandataire: Gevers & Orès
(86) Numéro de dépôt international: PCT/IB2012/055149
(87) Numéro de publication internationale: WO 2013/046144

(56) Documents cités:
- EP-A1- 1 281 320
- EP-A2- 0 424 845
- WO-A1-03/013250
- US-A1- 2005 214 386

## Description

### Domaine de l'invention

La présente invention concerne une méthode de décontamination de surfaces contaminées par un agent pathogène, notamment le prion.

### Arrière-plan technique

Du fait de l'exposition de la population au nouveau variant de la maladie de Creutzfeldt-Jakob (vMCJ) et devant l'impossibilité de caractériser le risque de transmission de l'agent responsable de cette maladie pour tout patient, des protocoles de décontamination efficaces vis-à-vis des prions ont dû être appliqués en milieu chirurgical.

Dans ce cadre, si pour les souches de prion liées aux cas de MCJ sporadiques, le risque se limite principalement aux instruments de neurochirurgie, dans le cas du vMCJ, les tissus lymphoïdes (ganglions, rate, amygdales et formations lymphoïdes de l'intestin) et le sang sont infectieux et généralisent donc le problème à l'ensemble des dispositifs médicaux, et notamment aux endoscopes. Ainsi, s'agissant notamment de ces derniers, il est nécessaire de les décontaminer entre deux actes d'endoscopie pour éviter des contaminations croisées de patients.

Par ailleurs, les sous-produits animaux (SPA) en provenance des abattoirs et des établissements d'équarrissage représentent des quantités très importantes de matières premières (près de 3.000.000 tonnes par an en France, 15.000.000 tonnes par an en Europe). Le traitement de ces sous-produits animaux conduit à la production de graisses animales (près de 500.000 tonnes par an en France, 2.500.000 tonnes par an en Europe) d'une part, et de protéines animales transformées (PAT - précédemment appelées farines animales - près de 700.000 tonnes par an en France, 3.500.000 tonnes par an en Europe) sous la forme de farines, d'autre part.

Avant les années 1990, ces dernières étaient principalement distribuées aux animaux de rente en tant que complément protéique. A l'époque, l'incapacité des procédés de fabrication de ces farines animales à inactiver les Agents transmissibles Non Conventionnels (ATNC), ou prions, a été à l'origine de la contamination de plusieurs centaines de milliers de bovins par l'agent de l'Encéphalopathie Spongiforme Bovine (ESB). Par la suite, la transmission de cette souche de prion du bovin à l'Homme (variante de la Maladie de Creutzfeldt-Jakob) a enclenché deux crises de santé publique et animale en France et en Europe en 1996 puis en 2000.

Au début des années 2000, l'introduction des tests de dépistage rapide a mis en évidence la persistance de l'épizootie en France malgré les mesures prises, et révélé l'existence de cas d'ESB dans l'ensemble de l'Europe, y compris dans des pays considérés indemnes jusqu'alors.

Les industriels qui ont à charge de collecter les SPA et les produits transformés correspondants (graisses et protéines), doivent ainsi dédier des véhicules de transport aux différentes catégories (camions citerne ou benne). Cette contrainte entraîne des retours à vide, les véhicules étant indisponibles pour des transports d'autre nature. Par ailleurs, il n'existe pas de moyen de décontamination applicable en pratique pour une réaffectation définitive des véhicules pour lesquels le besoin se réduit (notamment les véhicules dédiés au transport de sous-produits et de produits présentant un risque potentiel lié au prion). En conséquence l'immobilisation de ces moyens induit un surcoût pour les industriels et impacte directement le coût du traitement des SPA en général.

Les prions sont réputés pour persister pendant plusieurs années dans le milieu extérieur tout en conservant leur caractère infectieux (Brown and Gajdusek, 1991; Georgsson *et al.*, 2006). Ils sont également réputés présenter un large spectre de résistance vis-à-vis de nombreux procédés physico-chimiques classiquement utilisés dans le domaine de l'élimination et l'inactivation des agents biologiques. Par exemple, l'application de peroxyde d'hydrogène à 3% pendant une heure est inefficace. De fait, sur la base d'études réalisées sur des suspensions de prion, seules des méthodes drastiques sont réputées efficaces vis-à-vis de ces agents.

Ainsi, l'O.M.S. (W.H.O., 1999) recommande les traitements suivants, par ordre croissant d'efficacité :
1) Autoclavage à 134°C pendant 18 minutes.
2) Immersion pendant 1 heure dans une solution d'hydroxyde de sodium 1N à température ambiante.
3) Immersion pendant 1 heure dans une solution d'hypochlorite de sodium à 2 % de chlore actif à température ambiante.
4) Immersion dans une solution d'hydroxyde de sodium 1N et portée à ébullition pendant 10 minutes.
5) Immersion pendant 1 heure dans une solution d'hydroxyde de sodium 1N ou une solution d'hypochlorite de sodium à 2 % de chlore actif à température ambiante. Rinçage puis autoclavage à sec, à 121°C (autoclave à déplacement de gravité) ou 134°C (autoclave à charge poreuse) pendant 1 heure.
6) Immersion pendant 1 heure dans une solution d'hydroxyde de sodium 1N ou une solution d'hypochlorite de sodium à 2 % de chlore actif à température ambiante. Transfert dans l'eau et autoclavage à 121°C pendant 1 heure.
7) Immersion dans une solution d'hydroxyde de sodium 1N et autoclavage à 121°C pendant 30 minutes.

Du fait de leur caractère agressif particulièrement marqué, ces protocoles de décontamination recommandés par l'OMS impliquent d'utiliser du matériel chirurgical supportant ces traitements, en particulier du matériel autoclavable, ou à défaut de le remplacer par son équivalent à usage unique. Toutefois, cela s'avère impossible pour certains matériels, comme les endoscopes, qui sont complexes et coûteux et dont la fragilité les rend non-stérilisables thermiquement. En outre, il n'est pas possible d'appliquer les protocoles les plus efficaces, qui impliquent un autoclavage, à des matériels qui ont un volume important comme les véhicules de transport de farines. En outre, ces derniers résistent mal à la corrosion induite par des concentrations importantes de soude, de potasse ou d'hypochlorite de sodium.

Afin de tenter de résoudre ces problèmes, des méthodes n'impliquant pas d'autoclavage, faisant par exemple appel à des solutions moins agressives, ont été développées.

On connaît aussi les documents EP 1 281 320 A2 et US 2005/0214386 A1, qui décrivent des solutions différentes pour la désinfection des surfaces.

On peut citer, à ce titre, la solution commerciale HAMO 100 PID (Steris) qu'il est recommandé de laisser agir 15 minutes à 43°C. Toutefois, l'efficacité de cette solution est discutée (Fichet *et al.*, 2004 ; Hedgeworth *et al.*, 2011). En outre, sa composition n'est pas publique, ce qui ne permet pas de s'assurer de son absence d'agressivité vis-à-vis de surfaces à traiter.

Il reste donc à trouver des méthodes alternatives et efficaces de décontamination de surfaces susceptibles d'être contaminées par un agent pathogène, notamment un prion, moins agressives que celles existantes.

### Description de l'invention

La présente invention découle de la mise en évidence inattendue, par les inventeurs, qu'il est possible de décontaminer des surfaces contaminées par le prion ou par des spores de *Bacillus subtilis* avec une efficacité comparable à celles de l'état de la technique en utilisant une solution tamponnée modérément alcaline puis éventuellement une solution modérément oxydante.

Avantageusement, bien que de même nature (solution alcaline ou oxydante), les solutions employées selon l'invention sont moins concentrées que les solutions de l'état de la technique, ce qui leur confère un caractère moins agressif et permet de préserver les surfaces traitées. En outre, leur prix de revient est inférieur ou égal à celui des produits couramment utilisés.

L'invention repose sur un procédé de décontamination en deux étapes, correspondant, dans une première étape, à une déstabilisation réversible ou irréversible des agents pathogènes, dont les prions, suivie, dans une deuxième étape, d'une élimination et/ou inactivation irréversible. De manière intéressante, l'inversion des étapes peut rendre le procédé inefficace.

Sans vouloir être limité à une théorie particulière, les inventeurs pensent que la première étape permet de fragiliser ou de détruire les protéines de l'agent pathogène et de favoriser l'hydratation de la souillure organique incorporant l'agent pathogène, en utilisant une solution aqueuse alcaline. Cette première étape conduirait donc à une déstabilisation réversible, voire irréversible, des protéines associées à l'agent pathogène. Par ailleurs, cette étape peut également conduire à l'élimination des souillures organiques et des biofilms. La seconde étape, basée sur une solution oxydante, permet la destruction du potentiel infectieux des éventuels agents pathogènes fragilisés résiduels, qui sont traitées à cœur. Ainsi, la première étape permet de potentialiser les effets de la deuxième étape.

Ainsi, la présente invention concerne un procédé de décontamination d'une surface susceptible d'être contaminée, ou contaminée, par un agent pathogène tel que défini dans la revendication 1.

Une « surface » selon l'invention peut être de tout type susceptible d'avoir été en contact avec un agent pathogène. Toutefois, de manière préférée, la surface selon l'invention appartient à un instrument chirurgical, tel qu'un endoscope, notamment susceptible d'avoir servi, ou ayant servi, sur un individu susceptible d'être contaminé, ou étant contaminé, par un agent pathogène, ou à un véhicule de transport de sous-produits animaux transformés ou non, notamment susceptibles d'être contaminés par un agent pathogène. Comme on l'entend ici, les sous-produits animaux, transformés ou non, englobent, notamment, les carcasses animales, notamment de bovins. De préférence, la surface selon l'invention n'est pas constituée d'une partie d'un animal ou d'un être humain vivant, telle que la peau ou le tégument.

Comme on l'entend ici, la « décontamination d'une surface susceptible d'être contaminée par un agent pathogène » ou la « décontamination d'une surface contaminée par un agent pathogène » désigne la réduction de la quantité d'agents pathogènes éventuellement présents sur la surface. La réduction peut être due à un décrochage de l'agent pathogène de la surface et/ou à une destruction de l'agent pathogène ; on préfère, toutefois, selon l'invention, qu'il s'agisse d'une destruction de l'agent pathogène. En particulier, la « décontamination » selon l'invention consiste préférablement à réduire d'au moins 80%, 90%, 95%, 99%, 99,9%, 99,99%, ou 99,999% la quantité d'agent pathogène présent sur la surface selon l'invention. Comme cela apparaitra clairement à l'homme du métier la décontamination selon l'invention est de préférence conduite *in vitro*, c'est-à-dire qu'elle n'est pas appliquée au corps humain ou animal.

Les « agents pathogènes » selon l'invention peuvent être de tout type et sont notamment sélectionnés dans le groupe constitué d'un prion, d'un virus, d'une bactérie, d'un champignon, d'un protozoaire, et d'une spore de champignon ou de bactérie. De préférence, l'agent pathogène selon l'invention est un prion, une spore de bactérie, notamment du genre *Bacillus*, ou une mycobactérie. De manière particulièrement préférée, l'agent pathogène selon l'invention est un prion. Lorsque l'agent pathogène selon l'invention est un prion, la protéine constitutive du prion, ou PrP, se trouve essentiellement sous sa forme résistante, pathogène, PrPres.

Une « solution » selon l'invention est une solution aqueuse. La première solution selon l'invention, également nommée solution alcaline ou solution déstabilisante, a un pH supérieur ou égal à 9,5 et inférieur à 13. De préférence, le pH de la première solution est inférieur ou égal à 12,5, ou 12. De préférence également, le pH de la première solution est supérieur ou égal à 10,5, 11, ou 11,5. Comme on l'entend ici, le pH est mesuré à 20°C et à la pression atmosphérique. Comme cela apparaitra clairement à l'homme du métier, la première solution selon l'invention ne contient pas d'agent oxydant selon l'invention.

Une « base » selon l'invention désigne un composé susceptible de capter un proton (H⁺) et/ou de libérer un ion hydrogénate (OH⁻) dans une solution qui la contient. Une base est dite faible si, lorsqu'elle est ajoutée à une solution, elle capte significativement moins d'ions H⁺ et/ou qu'elle génère significativement moins d'ions OH⁻ que la quantité de base qui est ajoutée à la solution. Ainsi, la base faible selon l'invention doit bien être distinguée d'une base forte, telle que la potasse (KOH) ou la soude (NaOH). Par ailleurs, la base faible selon l'invention a un pKa compris entre 8 et 13, c'est-à-dire supérieur ou égal à 8 et inférieur ou égal à 13. De préférence, le pKa est inférieur ou égal à 12,5, 12, 11,5 ou 11. De préférence également, le pKa est supérieur ou égal à 8,5, 9, 9,5, ou 10. Comme on l'entend ici, la base faible selon l'invention peut avoir une ou plusieurs fonctions basiques. Lorsque la base faible selon l'invention comprend plusieurs fonctions basiques, le pKa selon l'invention est celui d'au moins l'une d'entre elles. Comme cela apparaitra clairement à l'homme du métier les valeurs de pKa selon l'invention sont de préférence celles mesurées dans les conditions de laboratoire de température et de pression, notamment à une température de 20°C à 25°C et à la pression atmosphérique, en particulier à une pression d'environ 1 bar.

La concentration de la base faible selon l'invention dans la solution est d'au moins 5.10⁻³ mol.l⁻¹, de préférence au moins 10⁻², 2.10⁻², 3.10⁻², 4.10⁻², 5.10⁻², 6.10⁻², 7.10⁻², 8.10⁻², 9.10⁻², ou 10⁻¹ mol.l⁻¹. De préférence également, la concentration de la base faible selon l'invention est d'au plus 1 mol.l⁻¹ ou 0,5 mol.l⁻¹.

Comme on l'entend ici, la concentration de la base faible désigne la concentration de la totalité des formes acido-basiques de la base faible dans la première solution. Par exemple, lorsque la base faible est un sel de carbonate, la concentration de la base faible dans la solution désigne la somme du nombre de moles de CO₃²⁻, de HCO₃⁻, et de H₂CO₃ dans la solution rapporté au volume de la solution ; pratiquement, étant donné le pH de la première solution, le nombre de moles de H₂CO₃ pourra toutefois être négligé.

La base faible selon l'invention peut être de toute nature. Toutefois, on préfère que la base faible selon l'invention soit un sel de carbonate, notamment du carbonate de sodium ou du carbonate de potassium. Comme on l'entend ici, un sel de carbonate désigne un sel de CO₃²⁻, un sel de HCO₃⁻, ou une combinaison des deux. Un sel de carbonate selon l'invention se dissocie de manière essentiellement complète dans la première ou la deuxième solution selon l'invention.

L'homme du métier sait parfaitement préparer une solution d'une concentration donnée d'une base faible à un pH particulier. Selon la base faible et le pH visé, on peut ajouter à la solution la base faible sous sa forme basique et/ou acide (c'est-à-dire ayant fixé un proton) et éventuellement ajuster le pH par l'ajout d'une base forte, telle que la potasse (KOH) ou la soude (NaOH). Ainsi, lorsque la base faible selon l'invention est un sel de carbonate, la première solution peut être préparée en ajoutant du Na₂CO₃, ou du K₂CO₃, et/ou du NaHCO₃, ou du KHCO₃, et éventuellement en ajustant le pH à l'aide de potasse et/ou de soude.

Comme on l'entend ici, un agent oxydant désigne tout agent susceptible de capter un électron. De préférence, l'agent oxydant selon l'invention est capable de générer une espèce réactive de l'oxygène. Selon un mode de réalisation préféré de l'invention, l'agent oxydant selon l'invention est halogéné. Plus préférablement encore, l'agent oxydant est sélectionné dans le groupe constitué d'un sel d'hypochlorite (HOCl), tel que l'hypochlorite de sodium, d'un sel de dichloro isocyanurate, tel que le dichloro isocyanurate de sodium (DCCNa), du peroxyde d'hydrogène (H₂O₂), et d'un peracide, tel que l'acide peracétique (APA). De manière particulièrement préférée, l'agent oxydant selon l'invention est un sel d'hypochlorite ou de dichloro isocyanurate. De manière particulièrement préférée également, l'agent oxydant selon l'invention est l'APA.

L'agent oxydant selon l'invention est contenu dans la solution à une concentration d'au moins 50 ppm, de préférence au moins 75 ppm, 100 ppm, 500 ppm, 1000 ppm, ou 1500 ppm. De préférence également, l'agent oxydant selon l'invention est contenu dans la solution à une concentration d'au plus 10000 ppm, plus préférablement au plus 5000 ppm, et encore plus préférablement au plus 2000 ppm. Comme l'homme du métier le sait bien 1 ppm = 0,0001% en masse/masse.

On préfère particulièrement que la première solution ait un pH d'au moins 11, que la base faible selon l'invention soit un sel de carbonate à une concentration d'au moins 10⁻² mol.l⁻¹, de préférence au moins 4.10⁻² mol.l⁻¹, et que l'agent agent oxydant soit un sel d'hypochlorite ou de l'APA à une concentration d'au moins 150 ppm.

La durée de la première étape peut être ajustée par l'homme du métier. Toutefois, on préfère que la durée de la première étape soit d'au moins 1, 2, 3, 4 ou 5 minutes, de préférence au moins 10 ou 15 minutes, encore plus préférablement au moins 30 minutes. On préfère également, que la première étape ait une durée de moins de deux heures, plus préférablement de moins d'une heure.

La durée de la deuxième étape peut également être ajustée par l'homme du métier. Toutefois, on préfère que la durée de la deuxième étape soit d'au moins 1, 2, 3, 4 ou 5 minutes, de préférence au moins 10 ou 15 minutes, encore plus préférablement au moins 30 minutes. On préfère également, que la deuxième étape ait une durée de moins de deux heures, plus préférablement de moins d'une heure.

De préférence la première étape et/ou la deuxième étape est conduite à moins de 100°C, plus préférablement moins de 80°C, encore plus préférablement moins de 60°C. Plus généralement, on préfère que le procédé selon l'invention ne comprenne pas d'étape conduite à plus de 100°C, en particulier à plus de 80°C, et plus particulièrement à plus de 60°C. On préfère également que le procédé selon l'invention ne comprenne pas d'étape d'autoclavage. Ainsi, de manière particulièrement préférée le procédé selon l'invention est mis en œuvre à température ambiante. Comme cela apparaitra clairement à l'homme du métier la température ambiante peut varier en fonction de la région géographique dans laquelle il est mis en œuvre, de la saison ou de l'heure de la journée, notamment lorsque le procédé de l'invention est conduit en plein air. Ainsi, en hiver, le procédé selon l'invention pourra être mis en œuvre à des températures de moins de 10°C et en été à des températures de plus de 30°C. Dans une définition particulière selon l'invention, toutefois, l'expression « température ambiante » vise préférentiellement des températures de 18°C à 25°C.

Par ailleurs, on préfère également que la température de la première solution et/ou de la deuxième solution au moment de la mise en contact avec la surface soit d'au moins 20°C, plus préférablement d'au moins 30°C, et plus préférablement encore d'au moins 40°C. Cette température peut être maintenue au cours de la mise en contact ou bien être laissée décroitre.

De manière particulièrement préférée, la première étape est conduite pendant 5 min à 43°C ou pendant 15 min à température ambiante, notamment à une température de 18°C à 25°C.

La première étape et la deuxième étape selon l'invention peuvent être fractionnées, c'est-à-dire que ces étapes peuvent comprendre chacune plusieurs périodes de durées différentes, chacune des périodes pouvant être associée à une température particulière. Ainsi, à titre d'exemple, la première étape selon l'invention peut comprendre une première période de 5 min à 43°C, suivie d'une deuxième période de 10 min à température ambiante.

Par ailleurs, comme cela apparaitra clairement à l'homme du métier le procédé selon l'invention peut être répété au moins 2 fois, notamment au moins 3, 4, 5, 6, 7, 8, 9, ou 10 fois, de manière à former un cycle. Comme cela apparaitra clairement à l'homme du métier, chaque répétition du cycle pourra se faire à des températures et sur des durées différentes des autres répétitions du cycle.

En outre, la première et la deuxième étape du procédé selon l'invention peuvent être mises en œuvre consécutivement ou bien être séparées, par exemple par une étape de rinçage. On préfère toutefois que la première et la deuxième étape soient mises en œuvre consécutivement sans étape intermédiaire.

Par ailleurs, dans un mode de réalisation préféré du procédé selon l'invention, la première étape et/ou la deuxième étape comprend également la mise en contact de la surface avec au moins un détergent. En particulier, le détergent peut être présent dans la première et/ou la deuxième solution selon l'invention ; dans ce cas on préfère que le détergent ne modifie pas le pH de la première et/ou de la deuxième solution. Le détergent selon l'invention peut être de tout type. On préfère toutefois qu'il s'agisse d'un détergent non ionique ou anionique. De manière davantage préférée, le détergent selon l'invention est sélectionné dans le groupe constitué d'un octylphénol éthoxylé, tel que l'ifralan OP 11 ou le triton X-100 ; d'un alkylsulfate de sodium, notamment dans lequel le groupement alkyl est en C8-C20, tel que le laurylsulfate de sodium ou sodium dodécyl sulfate (SDS).

En outre, dans un autre mode de réalisation préféré du procédé selon l'invention, la première étape et/ou la deuxième étape comprend également la mise en contact de la surface avec au moins un agent chélatant. En particulier, l'agent chélatant peut être présent dans la première et/ou la deuxième solution selon l'invention ; dans ce cas on préfère que l'agent chélatant modifie pas le pH de la première et/ou de la deuxième solution. L'agent chélatant peut être de tout type. On préfère toutefois que l'agent chélatant soit sélectionné dans le groupe constitué du 1-hydroxy éthylidène-1,1-diphosphonate (HEDP), du méthylglycinediacétate (Trilon), l'éthylène diamine tétra-acétate (EDTA), et leurs sels.

De préférence, le détergent et l'agent chélatant sont présents dans la première ou la deuxième solution. La concentration du détergent et de l'agent chélatant dans la première ou la deuxième solution est de préférence d'au moins 0,001%, 0,0025%, 0,01%, ou 0,02% exprimé en masse/masse. Par ailleurs, la concentration du détergent et de l'agent chélatant dans la première ou la deuxième solution est de préférence d'au plus 0,5%, 1% ou 2% exprimé en masse/masse.

Le détergent et l'agent chélatant sont utiles pour améliorer la décontamination induite par la première et éventuellement la deuxième solution, notamment lorsque l'eau de la solution est de l'eau non désionisée, par exemple lorsqu'il s'agit d'eau ayant une dureté supérieure à 0°f, notamment supérieure à 10°f, 20°f, 30°f, 40°f, 50°f ou 60°f, en particulier lorsque la base faible selon l'invention est un sel de carbonate. De manière avantageuse, l'ajout du détergent et/ou de l'agent chélateur permet de limiter l'apport de base faible ou forte nécessaire pour que le pH de la première solution soit conforme à l'invention, notamment lorsque l'eau de la première et/ou de la deuxième solution est une eau présentant une dureté supérieure à 0°f.

De manière préférée également, l'osmolarité de la première solution et/ou de la deuxième solution selon l'invention est supérieure ou égal à 10⁻², 2.10⁻², 3.10⁻², 4.10⁻², 5.10⁻², 6.10⁻², 7.10⁻², 8.10⁻², 9.10⁻², ou 10⁻¹ osmol.l⁻¹. On préfère aussi que l'osmolarité de la première solution et/ou de la deuxième solution selon l'invention est d'au plus 1 osmol.l⁻¹ ou 0,5 osmol.l⁻¹.

Par ailleurs, d'une manière générale, l'homme du métier est à même d'adapter les différents paramètres décrits ci-dessus entre eux afin de favoriser la décontamination de la surface par le procédé selon l'invention. Ainsi, lorsqu'on souhaite maintenir un pH de la première solution à une valeur basse on peut, outre ajouter un détergent selon l'invention, également augmenter la concentration de la base faible selon l'invention, augmenter la température de la première étape ou bien augmenter sa durée. De manière semblable, lorsqu'on souhaite mettre en œuvre le procédé selon l'invention à des températures de l'ordre de 18°C-25°C, on pourra augmenter le pH de la première solution, augmenter la concentration de la base faible, ou bien la durée de la première étape.

### Description des figures

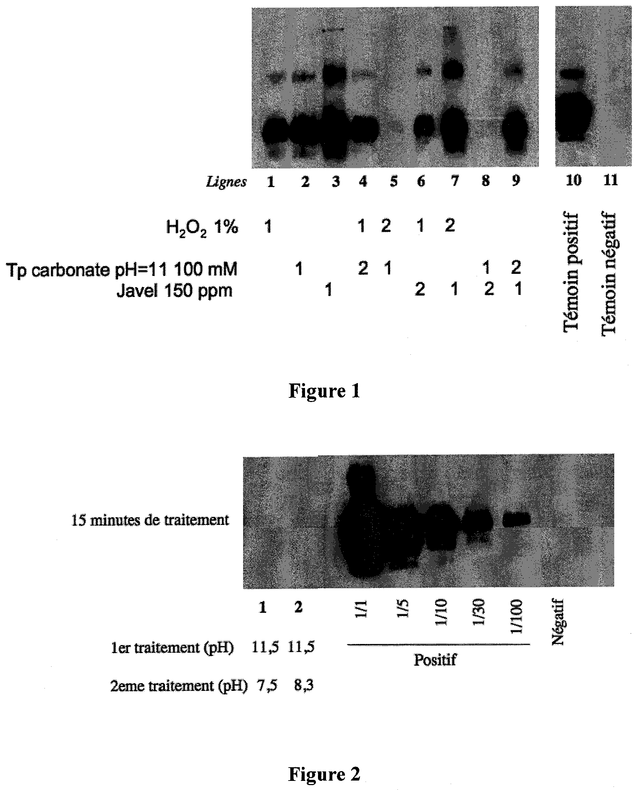

Figure 1 : Effet de la combinaison d'un traitement par un tampon carbonate pH=11 à 100 mM et d'un traitement oxydant par H₂O₂ 1% ou de l'eau de javel 150 ppm (les chiffres 1 et 2 indiquent l'ordre de passage dans les différents traitements) sur la quantité de PrPres restante après traitement de lames de verre présentant des homogénats de cerveau contaminés par PrPres.
Figure 2 : Effet de la combinaison d'un premier traitement par un tampon carbonate pH=11,5 à 10 mM et d'un deuxième traitement oxydant par l'acide peracétique à 1000 ppm sur la quantité de PrPres restante après traitement de lames de verre présentant des homogénats de cerveau contaminés par PrPres.

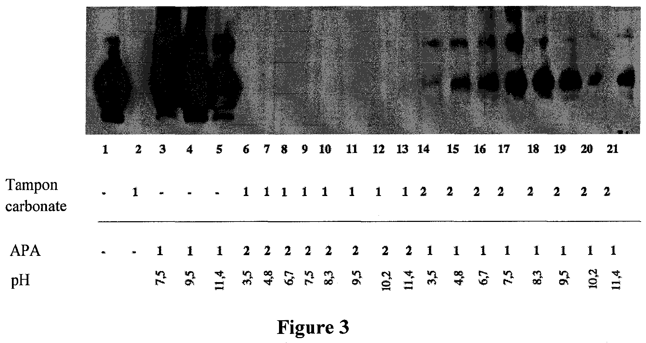

Figure 3 : Effet de la combinaison d'un premier traitement par un tampon carbonate pH=11 à 10 mM et d'un deuxième traitement oxydant par l'acide peracétique à 1000 ppm aux pH indiqué sur la quantité de PrPres restante après traitement de lames de verre présentant des homogénats de cerveau contaminés par PrPres (les chiffres 1 et 2 indiquent l'ordre de passage dans les différents traitements).

### Exemple

### 1.1 Introduction:

Les données expérimentales sont de deux types.

Une première étape a consisté à réaliser les essais sur des lames de verre avec des homogénats de cerveau pour simuler la contamination surfacique. Les analyses ont alors porté sur la souillure organique résiduelle et la présence de la forme résistance de la protéine du prion (PrPres), marqueur spécifique des encéphalopathies spongiformes subaiguës transmissibles (ESST). Des essais sur des suspensions liquides infectieuses ont été également réalisés afin d'identifier les procédés dont l'efficacité repose sur l'élimination de la contamination de surface (désorption).

Les traitements les plus efficaces sont ensuite validés *in vivo* par inoculation de surface (tiges métalliques) contaminées et traitées à des animaux de laboratoire, en accord avec le « protocole standard prion » préconisé par l'Agence Française de Sécurité Sanitaire des Produits de Santé (AFSSAPS).

Enfin, l'efficacité sporicide et mycobactéricide de certains traitements a été analysée sur des surfaces (lames de verre) contaminées par un homogénat de cerveau artificiellement contaminé avec une quantité connue de spore ou de mycobactérie.

### 1.2 Protocole in vitro:

### a) Préparation des sources infectieuses et des matrices

Les sources infectieuses correspondent à des homogénats de cerveau de hamster infecté par la souche 263K à 10% (poids/volume) dans une solution de PBS.

### b) Préparation des solutions pour traitement chimique et mesures du pH

Les tampons ont été préparés à 20°C ; les pH indiqués correspondent au pH de la solution.

Les tampons ont été préparés avec de l'eau osmosée produite à partir d'une station Millipore Elix de traitement d'eau. Différents tampons carbonate-bicarbonate (également nommés tampons carbonates) ont été préparés à partir de solutions de même molarité de bicarbonate de sodium (NaHCO₃) et de carbonate de sodium (Na₂CO₃), dans des proportions variables, et éventuellement par ajout de potasse (KOH), fournissant ainsi un panel de traitements présentant différents pH et différentes molarités (correspondant à la molarité des solutions de carbonate et de bicarbonate utilisées).

### c) Modélisation-miniaturisation du traitement

Vingt microlitres d'homogénat ont été étalés sur une lame de verre (5 cm x 2 cm). Les surfaces ont été séchées à l'air ambiant pendant une durée d'une heure. Les lames ont alors été rincées par immersion de 5 minutes en eau, afin de désorber la souillure organique mal fixée à la surface des lames. Les lames ont de nouveau été séchées pendant une heure, puis le traitement a été appliqué selon le protocole. Après rinçage final de 5 minutes en eau afin d'éliminer le traitement, les lames ont été séchées pendant une heure. La souillure organique résiduelle a alors été récupérée par grattage en présence de 120 µl d'eau, permettant ainsi de resolubiliser cette souillure organique pour les analyses *in vitro.*

### d) Analyse in vitro de la PrPres

Les souillures organiques resolubilisées ont été mélangées avec de l'homogénat de cerveau de hamster sain à 20% (poids/volume). La PrPres contenue dans ces échantillons a été purifiée au moyen des réactifs du test CEA-BioRad (Barret *et al.*, 2003), puis détectée par western blot, selon le protocole décrit précédemment (Lasmezas *et al.*, 1996). Après purification, les échantillons ont été resuspendus à l'aide d'un tampon Laemmli (Laemmli, 1970), et l'équivalent de 5 mg de cerveau a été déposé sur un gel de polyacrylamide et transféré sur une membrane de nitrocellulose (Schleicher & Schuell).

La révélation a été réalisée au moyen d'un anticorps monoclonal de souris reconnaissant spécifiquement la PrP de hamster (3F4). Un anticorps de chèvre anti-souris couplé à la peroxydase a été utilisé comme anticorps secondaire (Southern Biotechnology Associates), et l'immunoréactivité a été mise en évidence par chimiluminescence au moyen du kit ECL Amersham et détectée par autoradiographie.

### 1.3 Protocole in vivo:

### a) Dessin général de l'étude

Des tiges métalliques (Acier inoxydable « 316 », 5 mm x 0,16 mm pour les hamsters et 3 mm x 0,16 mm pour les souris) ont été contaminées artificiellement par incubation dans une suspension contenant une quantité connue d'agent de la tremblante adapté au hamster (souche 263K) ou d'agent de l'ESB adapté à la souris (souche 6PB1) respectivement (Zobeley *et al.*, 1999 ; Fichet *et al.*, 2004). Après séchage, les tiges ont été soumises à un des protocoles de nettoyage et/ou de désinfection étudiés.

Après décontamination, les tiges ont été inoculées individuellement à des animaux de laboratoire (hamster ou souris) par voie intra-cérébrale (i.c.), afin de mesurer l'éventuelle charge infectieuse résiduelle à la surface des tiges.

### b) Conditions d'hébergement des animaux

Les animaux sont hébergés dans une animalerie de haute sécurité de niveau 3. Chaque animal est identifié au moyen d'une puce électronique.

Les souris sont réparties au hasard dans des cages à raison de 8 ou 12 animaux par cage, et les hamsters à raison de 4 animaux par cage. Les litières sont changées régulièrement au moins une fois par semaine.

### c) Souches d'agent

La souche d'agent transmissible non conventionnel (ATNC) choisie pour la validation dans le modèle cricétidien est la souche de tremblante 263K, souche sélectionnée et stabilisée chez le hamster syrien et titrant environ 2.10¹⁰ DL50 par gramme de cerveau (Kimberlin and Walker, 1967; Adjou *et al.*, 1995). La période d'incubation de la maladie est en moyenne de 70 jours et la mort des animaux survient en moyenne 80 à 90 jours après l'inoculation par voie i.c. de 10⁷ fois la dose létale 50 (DL50).

La souche d'ATNC choisie pour la validation dans le modèle murin est une souche d'ESB 6PB1 titrant 10⁸ à 10⁹ DL50 par gramme de cerveau (Lasmézas *et al.*, 1996). Chez la souris C57B1/6, la période d'incubation de la maladie est en moyenne de 150 jours et la mort des animaux survient en moyenne 180 jours après l'inoculation par voie i.c. de 10⁵ DL50.

Les inocula positifs ont été préparés à partir de cerveaux de hamsters ou de souris respectivement infectées par les souches de tremblante 263K et d'ESB 6PB1, au stade terminal de la maladie. Les inocula négatifs ont été préparés à partir de cerveaux de hamsters et de souris sains. Les cerveaux ont été homogénéisés à 10 % (poids/volume) dans du PBS.

### d) Contamination artificielle des tiges

Les tiges métalliques ont été contaminées artificiellement.

Brièvement, les inocula positifs ont été préparés à partir de cerveaux de hamsters ou de souris respectivement infectées par les souches de tremblante 263K et d'ESB 6PB1, au stade terminal de la maladie. Les inocula négatifs ont été préparés à partir de cerveaux de hamsters et de souris sains. Les cerveaux ont été homogénéisés à 10 % (poids/volume) dans du PBS.

Les tiges métalliques ont alors été contaminées par incubation dans les homogénats correspondants pendant 1 heure à température ambiante.

Les tiges métalliques dépolies (Acier « 316 », 5 mm x 0,16 mm pour les hamsters et 3 mm x 0,16 mm pour les souris) ont été traitées par ultrasonication pendant 15 minutes dans une solution de triton X-100 à 2 %, rincées dans de l'eau distillée puis séchées à 37°C pendant 1 heure. Les tiges ont été incubées dans 200 µl d'homogénat à 10 % pendant 1 heure à température ambiante. Les tiges ont alors été séchées pendant 16 heures à température ambiante sous hotte à flux laminaire, pour permettre l'adsorption des agents infectieux à la surface des tiges.

La durée d'incubation de la maladie étant directement corrélée au titre infectieux de l'inoculum, les tiges contrôles ont été contaminées par incubation dans des dilutions croissantes de 10 en 10 d'inoculum positif dans de l'inoculum négatif, puis inoculées aux animaux. Les animaux sont suivis pour tracer la courbe reliant la moyenne des temps de survie à la charge infectieuse inoculée.

### e) Inoculation à l'animal et suivi des animaux

Les animaux anesthésiés ont été inoculés par voie intra-cérébrale (i.c.) à l'aide d'une seule tige, ou à l'aide de 50 µl de surnageant traité. Pour chaque traitement et chaque point des contrôles, 8 ou 12 animaux ont été inoculés.

Le titre infectieux est établi sur la base des dates de décès et du constat du stade terminal. L'examen clinique est relié à l'observation des signes pathognomoniques des encéphalopathies subaiguës spongiformes transmissibles (ESST).

Avant l'apparition des premiers signes cliniques dans les groupes témoins (jusqu'au 50^{e} jour pour les hamsters, 150^{e} jour pour les souris), les animaux de tous les groupes ont été observés cliniquement une fois par semaine.

A partir du 50^{e} jour pour les hamsters et 150^{e} jour pour les souris et jusqu'à la fin de l'étude, les examens cliniques sont pratiqués trois fois par semaine.

Les signes cliniques sont de cinq ordres :
- Troubles du comportement (excitabilité, troubles de la nidation et du toilettage),
- Perte du réflexe de positionnement visuel,
- Ataxie cérébelleuse, démarche anormale, prostration,
- Obésité puis amaigrissement,
- Paraplégie puis tétraplégie.

Les observations sont consignées après chaque examen clinique. Les signes cliniques et les décès sont enregistrés. Les animaux au stade terminal de la maladie sont euthanasiés pour limiter la souffrance.

En cas de mort des animaux inoculés avec les tiges traitées, la moyenne du temps de survie de ces groupes est comparée à la moyenne des temps de survie des animaux dans les groupes contrôles. Il est alors possible d'estimer la charge infectieuse résiduelle après traitement des tiges, et de déterminer ainsi le niveau de réduction apporté par ce traitement.

Douze mois après l'inoculation des hamsters (soit 4 fois le temps d'incubation de la maladie après inoculation par voie i.c. de 10⁷ DL50 pour la souche 263K) et dix-huit mois après l'inoculation des souris (soit 6 fois le temps d'incubation de la maladie après inoculation par voie i.c. de 10⁵ DL50 pour la souche 6PB1), tous les animaux survivants ont été euthanasiés. Les cerveaux de ces animaux ont été prélevés, et la quantité de PrPres éventuellement présente dans ces cerveaux a été estimée au moyen d'une technique biochimique (ELISA ou Western blot). Les cerveaux de certains animaux décédés au cours de l'expérimentation ont également été étudiés à l'aide de ces tests biochimiques.

### f) Détection de la PrPres au moyen d'une technique biochimique (ELISA / Western blot)

La détection de la PrPres a été réalisée par le test rapide TeSeE BioRad (concentration de la PrPres et détection par Elisa) adapté aux rongeurs, à partir d'un hémisphère cérébral homogénéisé à 20 % dans une solution glucosée à 5 % (Barret *et al.*, 2003). La valeur seuil correspond à 2,5 fois la densité optique (D.O.) moyenne des contrôles négatifs. Les échantillons présentant une D.O. inférieure à la valeur seuil sont considérés comme négatifs. Les échantillons présentant une D.O. supérieure à la valeur seuil sont considérés comme positifs.

Certains échantillons positifs ont été confirmés par western blot, selon le protocole décrit précédemment (Lasmezas *et al.*, 1996). Après purification, les échantillons sont resuspendus à l'aide d'un tampon Laemmli (Laemmli, 1970), et l'équivalent de 5 mg de cerveau a été déposé sur un gel de polyacrylamide et transféré sur une membrane de nitrocellulose (Schleicher & Schuell).

La révélation a été réalisée au moyen d'un anticorps monoclonal reconnaissant spécifiquement la PrP murine. Un anticorps de chèvre anti-souris couplé à la peroxydase est utilisé comme anticorps secondaire (Southern Biotechnology Associates). L'immunoréactivité a été mise en évidence par chimiluminescence au moyen du kit ECL Amersham et détectée par autoradiographie.

### 1.4 Résultats

### 1.4.1 Données expérimentales in vitro sur les prions

### a) Principe de l'invention

La **Figure 1** illustre le principe de la présente invention, à savoir l'efficacité d'une combinaison de deux traitements inefficaces seuls dont la séquence est déterminante.

On observe que l'immersion des lames contaminées pendant 5 minutes à température ambiante dans du peroxyde d'hydrogène à 1 % (ligne 1) ou de l'eau de javel à 150 ppm (ligne 3) ne modifie pas significativement la quantité apparente de PrPres par rapport au témoin positif (ligne 10). L'utilisation séquentielle de ces deux produits, quel qu'en soit le sens, n'est pas non plus efficace (ligne 6 et 7).

En revanche, tandis que l'immersion pendant 5 minutes en tampon carbonate-bicarbonate 100mM à pH=11 ne modifie pas non plus la quantité apparente de PrPres (ligne 2), l'utilisation de ce tampon suivie de l'application du peroxyde d'hydrogène (ligne 5) ou de l'eau de javel (ligne 8) réduit de façon significative la quantité de PrPres. A l'inverse, l'utilisation de cette combinaison de produits en sens inverse (lignes 4 et 9) est inefficace.

Des résultats similaires peuvent être obtenus également avec l'acide peracétique (**Figures 2** **et** **3**).

La **Figure 2** présente les résultats obtenus en croisant un traitement de déstabilisation (tampon carbonate-bicarbonate 10 mM, pH = 11,5) en présence d'un détergent non ionique (octylphénol éthoxylé, Ifralan OP-11) (0,1 %) et d'un chélateur (acide 1-hydroxy éthylidène-1,1-diphosphonique, ou HEDP) (0,1 %), avec deux traitements d'oxydation à base d'acide peracétique titrant 1000 ppm à pH 7,5 ou 8,3 (réalisés à température ambiante, ces deux traitements contiennent de l'ifralan à 0,02 %). La quantité apparente de PrPres est réduite de plus de 2 logs.

Un premier traitement de déstabilisation à l'aide d'un tampon carbonate 10 mM à pH 11 a été appliqué pendant 5 minutes à 43°C suivi de l'application de solutions d'acide peracétique 1000 ppm à différents pH (**Figure 3**). Lorsqu'il est appliqué en premier, ce traitement déstabilisant est efficace seul (ligne 2) et l'utilisation ultérieure de préparations à base d'acide peracétique inefficaces seules (lignes 3 à 5) ne modifie pas cette efficacité (lignes 6 à 13). En revanche, l'utilisation au préalable de l'acide peracétique empêche l'activité de la solution de déstabilisation (lignes 14 à 21) même lorsque ce traitement oxydant est réalisé à des pH supérieurs à 10 (lignes 20 et 21).

### b) Généralisation

Des mélanges, contenant ou non de l'ifralan à 1/1000 et du HEDP à 1/1000, ont été préparés et appliqués pendant 5 minutes à 43°C sur des lames contaminées. Les résultats sont présentés dans le **Tableau 1** suivant.

**Tableau 1 : Traitement de lames contaminées par le prion par des tampons carbonate-bicarbonate de différents pH, contenant ou non de l'ifralan et du HEDP.**

| Présence d'Ifralan + HEDP | | Oui | Oui | |
|---|---|---|---|---|
| Molarité du tampon carbonate | | 15^{a} | 20^{b} | 20^{c} |
| pH | | 11,29 | 11,45 | 11,74 |
| Sans rinçage | HClO | E | E | E |

| | | | | |
|---|---|---|---|---|
| E : efficace (pas de signal apparent de PrPres) | | | | |

D'autres mélanges, contenant ou non de l'ifralan à 1/1000 et du HEDP à 1/1000, ont été préparés et appliqués pendant 5 minutes à 43°C sur des lames contaminées, puis, éventuellement après rinçage, un traitement oxydant (DiChloro isoCyanurate de sodium (troclosène de sodium, DCCNa) à 150 ppm, Hypochlorite (HClO) à 150 ppm, Peroxyde d'hydrogène (H₂O₂) à 1%, ou rien) ou une simple application d'eau à été appliqué pendant 5 minutes à 43°C. Les résultats obtenus sont présentés dans les **Tableaux 2 à 4** qui suivent.

**Tableau 2 : Traitement de lames contaminées par le prion par des tampons carbonate-bicarbonate de différents pH, contenant ou non de l'ifralan et du HEDP, suivi par un traitement par HClO**

| **Premier traitement** | | | | | | | | | | | |
|---|---|---|---|---|---|---|---|---|---|---|---|
| Présence d'Ifralan + HEDP | | | Oui | Oui | Oui | | | Oui | Oui | | |
| Molarité du tampon carbonate | | 40^{d} | 10^{e} | 15^{f} | 10^{g} | 15^{h} | 10ⁱ | 15^{a} | 20^{b} | 15^{j} | 20^{c} |
| pH | | 10,43 | 10,5 | 10,84 | 10,92 | 11,13 | 11,2 | 11,29 | 11,45 | 11,67 | 11,74 |

| **Second traitement** | | | | | | | | | | | |
|---|---|---|---|---|---|---|---|---|---|---|---|
| Sans rinçage | HClO | E | E | E | E | E | E | E | E | E | E |
| Avec rinçage | HClO | E | E | E | E | E | E | E | E | E | E |

| | | | | | | | | | | | |
|---|---|---|---|---|---|---|---|---|---|---|---|
| E : efficace (pas de signal apparent de PrPres) | | | | | | | | | | | |

**Tableau 3 : Traitement de lames contaminées par le prion par des tampons carbonate-bicarbonate de différents pH, contenant ou non de l'ifralan et du HEDP, suivi par un traitement par DCCNa**

| Présence d'Ifralan + HEDP | | Oui | Oui | Oui | Oui | | |
|---|---|---|---|---|---|---|---|
| Molarité du tampon carbonate | | 15^{f} | 10^{g} | 15^{a} | 20^{b} | 15^{j} | 20^{c} |
| pH | | 10,84 | 10,92 | 11,29 | 11,45 | 11,67 | 11,74 |
| Sans rinçage | DCCNa | E | E | E | E | E | E |
| Avec rinçage | DCCNa | E | E | E | E | E | E |

| | | | | | | | |
|---|---|---|---|---|---|---|---|
| E : efficace (pas de signal apparent de PrPres) | | | | | | | |

**Tableau 4 : Traitement de lames contaminées par le prion par des tampons carbonate-bicarbonate de différents pH, contenant ou non de l'ifralan et du HEDP, suivi par un traitement par H₂O₂.**

| Présence d'Ifralan + HEDP | | Oui | | Oui | Oui | | |
|---|---|---|---|---|---|---|---|
| Molarité du tampon carbonate | | 10^{g} | 10ⁱ | 15^{a} | 20^{b} | 15^{j} | 20^{c} |
| pH | | 10,92 | 11,2 | 11,29 | 11,45 | 11,67 | 11,74 |
| Sans rinçage | H₂O₂ | E | E | E | E | E | E |
| Avec rinçage | H₂O₂ | E | E | E | E | E | E |

| | | | | | | | |
|---|---|---|---|---|---|---|---|
| E : efficace (pas de signal apparent de PrPres) | | | | | | | |

Les tampons carbonate utilisés dans les expériences dont les résultats sont rapportés dans les

**Tableaux 1 à 4 précédents ont été préparés comme suit (le tampon correspond aux lettres en exposant utilisées dans les tableaux) :**

| **Tampon** | **Na₂CO₃** | **NaHCO₃** | **KOH** |
|---|---|---|---|
| a | 15 mM (33,7%) | 15 mM (66,3%) | 15 mM |
| b | 20 mM (33,7%) | 20 mM (66,3%) | 20 mM |
| c | 20 mM (33,7%) | 20 mM (66,3%) | 20 mM |
| d | 40 mM (63,7%) | 40 mM (36,3%) | - |
| e | 10 mM (33,7%) | 10 mM (66,3%) | 5mM |
| f | 15mM (11,7%) | 15 mM (88,3%) | 15mM |
| g | 10 mM (63,7%) | 10 mM (36,3%) | 5mM |
| h | 15mM (11,7%) | 15 mM (88,3%) | 15mM |
| i | 10 mM (63,7%) | 10 mM (36,3%) | 5mM |
| j | 15 mM (33,7%) | 15 mM (66,3%) | 15mM |

En outre, l'effet de combinaisons additionnelles de tampons carbonate-bicarbonate, à différentes concentrations et différents pH, avec un ou plusieurs détergents (laurylsulfate de sodium (Sulfétal, Texapon)) et un agent chélatant (HEDP et méthylglycinediacétate (Trilon)) sur des lames contaminées par le prion ont été testés (**Tableau 5**).

**Tableau 5 : Traitement de lames contaminées par le prion par des tampons carbonate-bicarbonate de différents pH, contenant ou non du sulfétal, du texapon, du HEDP et/ou du trilon**

| Molarité du tampon carbonate | | | 0 | | | 20 | | | 10 | | |
|---|---|---|---|---|---|---|---|---|---|---|---|
| Sulfétal | Texapon | HEDP/Trilon | pH | Souillure | PrPres | pH | Souillure | PrPres | pH | Souillure | PrPres |
| - | - | - | 6,62 | +++ | I | | | | | | |
| - | 0,01% | - | 8,68 | +++ | I | | | | | | |
| 0,01% | - | - | 8,72 | +++ | I | | | | | | |
| 0,01% | 0,001% | - | 8,65 | +++ | I | | | | | | |
| 0,1% | - | - | 8,80 | +++ | I | | | | | | |
| 0,1% | 0,01% | - | 8,75 | +++ | I | | | | | | |
| - | - | HEDP 0,1% | 9,40 | ++ | I | | | | | | |
| 0,01% | - | HEDP 0,1% | 9,25 | ++ | D | | | | | | |
| 0,01% | 0,001% | HEDP 0,1% | 9,30 | + | D | | | | | | |
| 0,1% | - | HEDP 0,1% | 9,22 | +/- | I | | | | 10,44 | - | E |
| 0,1% | 0,01% | HEDP 0,1% | 9,32 | +/- | D | | | | 10,40 | - | E |
| - | - | Trilon 0,1% | 9,75 | ++ | I | | | | | | |
| - | 0,01% | Trilon 0,1% | 9,78 | +++ | I | | | | | | |
| 0,1% | - | Trilon 0,1% | 9,78 | +/- | D | | | | 10,47 | - | E |
| 0,1% | 0,01% | Trilon 0,1% | 9,76 | +/- | D | 10,201 | - | E | 10,46 | - | E |

| Molarité du tampon carbonate | | | 25 | | | 10 | | | | | |
|---|---|---|---|---|---|---|---|---|---|---|---|
| Sulfétal | Texapon | HEDP/Trilon | pH | Souillure | PrPres | pH | Souillure | PrPres | | | |
| - | - | - | | | | 11,40 | +/- | E | | | |
| - | 0,01% | - | | | | | | | | | |
| 0,01% | - | - | | | | | | | | | |
| 0,01% | 0,001% | - | | | | | | | | | |
| 0,1% | - | - | 11,01 | +/- | E | 11,39 | | E | | | |
| 0,1% | 0,01% | - | 11,02 | - | E | 11,41 | - | E | | | |
| - | - | HEDP 0,1% | | | | 11,57 | - | E | | | |
| 0,01% | - | HEDP 0,1% | | | | 11,55 | - | E | | | |
| 0,01% | 0,001% | HEDP 0,1% | | | | 11,54 | - | E | | | |
| 0,1% | - | HEDP 0,1% | | | | 11,60 | - | E | | | |
| 0,1% | 0,01% | HEDP 0,1% | | | | 11,52 | - | E | | | |
| - | - | Trilon 0,1% | | | | 11,62 | - | E | | | |
| - | 0,01% | Trilon 0,1% | | | | 11,62 | - | E | | | |
| 0,1% | - | Trilon 0,1% | 11,10 | - | E | 11,63 | - | E | | | |
| 0,1% | 0,01% | Trilon 0,1% | 11,09 | - | E | 11,62 | - | E | | | |

| | | | | | | | | | | | |
|---|---|---|---|---|---|---|---|---|---|---|---|
| I : inefficace D : déstabilisant (diminution du signal apparent de PrPres) E : efficace (pas de signal apparent de PrPres) | | | | | | | | | | | |

### 1.4.2 Données expérimentales in vitro de sporicidie

L'effet des différents traitements (tampon carbonate-bicarbonate suivi d'un traitement à l'acide peracétique) sur les spores a été étudié, en prenant comme modèle *Bacillus subtilis* (**Tableau 6**)

Une suspension d'un nombre connu de spore a été mélangée avec un homogénat de cerveau, afin de travailler avec le même type de matrice que celle utilisée pour les études prion. Après exposition aux différents traitements, la souillure organique résiduelle a été récupérée et mise en culture. La présence résiduelle de souillure organique après application du traitement a été évaluée, visuellement et par mesure de la densité optique de la souillure organique après resolubilisation.

**Tableau 6 : Sporicidie de différentes combinaisons (les facteurs de réduction en termes de sporicidie sont exprimés en log).**

| | | | **SANS RINCAGE** | | |
|---|---|---|---|---|---|
| **Premier traitement (15 minutes 25°C)** | | **Second traitement (5 minutes 25°C)** | **Souillure résiduelle** | | **Réduction spores** |
| | **pH** | | **Visuel** | **D.O.** | |
| H2O | | H2O | +++ | 1,297 | 0 |
| KOH 10mM | 12,37 | H2O | - | 0,088 | 1 |
| KOH 5mM | 11,73 | H2O | + | 0,050 | 0 |
| H2O | | APA | +++ | 1,468 | 4 |
| KOH 10mM | 12,37 | APA | - | 0,098 | 5 |
| KOH 5mM | 11,73 | APA | + | 0,152 | 5 |
| Tampon bicarbonate 10mM + KOH 10 mM | 10,86 | APA | ++ | 0,448 | >6 |
| Tampon bicarbonate 5mM + KOH 10 mM | 11,70 | APA | +/++ | 0,111 | >6 |
| Tampon carbonate (33,7%)-bicarbonate (66,3%) 10mM + KOH 10mM | 11,37 | APA | +/++ | 0,239 | >6 |

On observe que l'association de ces deux traitements permet d'obtenir une efficacité du protocole complet vis-à-vis des spores des prions, de manière semblable à ce qui est obtenu avec les prions. Des résultats semblables sont obtenus avec un rinçage entre les deux traitements.

### 1.4.3 Données expérimentales in vivo vis-à-vis des prions

Les tiges qui ont été utilisées dans ces études ont subi au préalable à leurs contaminations des traitements drastiques de dépolissage (combinaison de traitements fortement basiques et thermiques), ayant pour but de les vieillir artificiellement d'une part, et de générer des anfractuosités d'autre part, de façon à se trouver dans un scénario de surface particulièrement difficile à décontaminer.

Une expérience *in vivo* est en cours (modèle hamster inoculé avec la souche 263K et souris Swiss inoculée avec la souche vMCJ), impliquant un traitement par des tampons carbonate-bicarbonate.

### REFERENCES

- Barret, A., Tagliavini, F., Forloni, G., Bate, C., Salmona, M., Colombo, L., De Luigi, A., Limido, L., Suardi, S., Rossi, G., Auvré, F., Adjou, K.T., Salés, N., Williams, A., Lasmézas, C. and Deslys, J.P. (2003) Evaluation of quinacrine treatment for prion diseases. J. Virol. 77 : 8462-8469.
- Bauman, P.A., Lawrence, L.A., Biesert, L., Dichtelmüller, H., Fabbrizzi, F., Gajardo, R., Gröner, A., Jorquera, J.I., Kempf, C., Kreil, T.R., Von Heugen, I., Pifat, D.Y., Petteway, S.R. and Cai, K. (2006) Critical factors influencing prion inactivation by sodium hydroxide. Vox Sanguinis 91 : 34-40.
- Brown, P., Rohwer, R.G., Green, E.M. and Gajdusek, D.C. (1982a) Effect of chemicals, heat and histopathologic processing on high-infectivity hamster-adapted scrapie virus. J. Infect. Dis. 145 : 683-687.
- Brown, P., Gibbs, CJ, Amyx, HL, et al. (1982b) chemical disinfection of Creutzfeldt-Jakob disease virus. N Engl J Med 306: 1279-1282.
- Brown, P., Rohwer, R.G. and Gajdusek, D.C. (1986) Newer data on the inactivation of scrapie virus or Creutzfeldt-Jakob disease virus in brain tissue. J. Infect. Dis. 153 : 1145-1148.
- Brown, P., Liberski, P.P., Wolff, A. and Gajdusek, D.C. (1990) Résistance of scrapie infectivity to steam autoclaving after formaldehyde fixation and limited survival after ashing at 360°C : practival and theoretical implications. J. Infect. Dis. 161 : 467-472.
- Brown, P. and Gajdusek, D.C. (1991) Survival of scrapie virus after three years' internment. Lancet 337 : 269-270.
- Brown, P., Meyer, R., Cardone, F. and Pocchiari, M. (2003) Ultra-high-pressure inactivation of prion infectivity in processed meat: a practical method to prevent human infection. Proc. Natl. Acad. Sci. USA 100 : 6093-6097.
- Brown, S.A., Merritt, K., Woods, T.O. and Busick D.N. (2005) Effects on instruments of the WHO-recommended protocols for decontamination after possible exposure to transmissible spongiform encephalopathy-contaminated tissue. J. Biomed. Mater. Res. Part B : Appl. Biomater. 72B : 186-190.
- Castilla, J., Gutiérrez Adan, A., Brun, A., Pintado, B., Ramirez, M.A., Parr, B., Doyle, D., Rogers, M., Salguero, F.J., Sanchez, C., Sanchez-Vizcaino, J.M. and Torres, J.M. (2003) Early détection of PrPres in BSE-infected bovine PrP transgenic mice. Arch. Virol. 148 : 677-691.
- Décision Commission Européenne 6/449/CE (18 Juillet 1996) Décision de la Commission relative à l'agrément de systèmes de traitement thermique de remplacement pour la transformation de déchets animaux au regard de l'inactivation des agents de l'encéphalopathie spongiforme. Official Journal of the European Communities L184 : 43.
- Directive Européenne 90/667/CE (27 novembre 1990) Règles sanitaires relatives à l'élimination et à la transformation de déchets animaux à leur mise sur le marché et à la protection contre les agents pathogènes des aliments pour animaux d'origine animale ou à base de poisson, et modifiant la Directive 90/425/CEE. Official Journal of the European Communities L363 : 51-60.
- Dickinson, A.G. and Taylor, D.M. (1978) Résistance of scrapie agent to decontamination. N. Engl. J. Med. 299 : 1413-1414.
- Ernst, D.R. and Race, R.E. (1993) Comparative analysis of scrapie agent inactivation methods. J. Virol. Methods 41 : 193-202.
- Féraudet, C., Morel, N., Simon, S., Volland, H., FrobeTA, Y., Créminon, C., Vilette, D., Lehmann, S. and Grassi, J. (2005) Screening of 145 anti-PrP monoclonal antibodies for their capacity to inhibit PrPsc replication in infected cells. J. Biol. Chem. 280 : 11247-11258.
- Fernie, K., Steele, P.J., Taylor, D.M. and Somervilles, R.A. (2007) Comparative studies on the thermostability of five strains of transmissible-spongiform-encephalopathy agent. Biotechnol. Appl. Biochem. 47 : 175-183.
- Fichet, G., Comoy, E., Duval, C., Antloga, K., Dehen, C., Charbonnier, A., McDonnell, G., Brown, P., Lasmézas, C.I. and Deslys, J.P. (2004) Novel methods for disinfection of prion-contaminated medical devices. The Lancet 364 : 521-526.
- Fichet, G., Antloga, K., Comoy, E., Deslys, J.P. and McDonnell, G. (2007a) Prion inactivation using a new gaseous hydrogen peorxide stérilisation process. J. Hosp. Infect. 67 : 279-287.
- Fichet, G., Comoy, E., Dehen, C., Challier, L., Antloga, K., Deslys, J.P. and McDonnell, G. (2007b) Investigations of a prion infectivity assay to evaluate methods of decontamination. J. Microbiol. Meth. 70 : 511-518.
- Georgsson, G., Sigurdarson, S. and Brown, P. (2006) Infectious agent of sheep scrapie may persist in the environment for at least 16 years. J. Gen. Virol. 87 : 3737-3740.
- Hunter, G.D. and Millson, G.C. (1964) Studies on the heat stability and chromatographic behaviour of the scrapie agent. J. Gen. Microbiol. 37 : 251-258.
- Hedgeworth J.A. et al. (2011) A standardized comparison of commercially available prion decontaminating reagent using the Standard Steel-Binding Assay. J. Gen. Virol. 92 : 718-726
- Käsermann, F. and Kempf, C. (2003) Sodium hydroxide renders the prion protein PrPsc ensitive to proteinase K. J. Gen. Virol. 84: 3173-3176.
- Kimberlin, R.H. and Walker, C.A. (1977) Characteristics of a short incubation model of scrapie in the golden hamster. J. Gen. Virol. 34 : 295-304.
- Kimberlin, R.H., Walker, C.A., Millson, G.C., Taylor, D.M., Robertson, P.A., Tomlinson, A.H. and Dickinson, A.G. (1983) Disinfection studies with two strains of mouse-passaged scrapie agent. J. Neurol. Sci. 59 : 355-369.
- Læmmli, U.K. (1970) Cleavage of structural proteins during the assembly of bacteriophage T4. Nature 227 : 680-685.
- Lasmézas, C.I., Deslys, J.P., Demaimay, R., Adjou, K.T., Hauw, J.J. and Dormont, D. (1996) Strain specific and common pathogenic events in murine models of scrapie and bovine spongiform encephalopathy. J. Gen. Virol. 77 : 1601-1609.
- Lawson, V.A., Stewart, J.D. and Masters, C.L. (2007) Enzymatic detergent treatment protocol that reduces protease-resistant prion protein load and infectivity from surgical-steel monofilaments contaminated with a human-derived prion strain. J. Gen. Virol. 88 : 2905-2914.
- Lignes Directrices relatives aux demandes d'approbation de nouveaux modes d'élimination ou d'utilisation des sous-produits animaux en application du règlement (CE) N° 1774/2002.
- McLeod, A.H., Murdoch, H., Dickinson, J., Dennis, M.J., Hall, G.A., Buswell, C.M., Carr, J., Taylor, D.M., Sutton, J.M. and Raven, D.H. (2004) Proteolytic inactivation of the bovine spongiform encephalopathy agent. Biochem. Biophys. Res. Comm. 317: 1165-1170.
- Mould, D.L. and Dawson, A. (1970) The response in mice to heat treated scrapie agent. J. Comp. Pathol. 80 : 595-600.
- Opinion of the Scientific Panel on Biological Hazards of the EFSA (2005) Quantitative risk assessment of the animal BSE risk posed by meat and bone meal with respect to the residual BSE risk. EFSA Journal 257 : 1-30.
- Règlement (CE) n°1774/2002 du Parlement Européen et du Conseil (03 Octobre 2002) établissant des règles sanitaires applicables aux sous-produits animaux non destinés à la consommation humaine. Official Journal of European Communities L273 : 1-95. http://eur-lex.europa.eu/LexUriServ/LexUriServ.do?uri=OJ:L:2002:273:0001:0095:FR:PDF
- Schreuder, B.E., GeeTAsma, R.E., Van Keulen, L.J., Van Asten, J.A., Enthoven, P., Oberthür, R.C., de Koeijer, A.A. and Osterhaus, A.D. (1998) Studies on the efficacy of hyperbaric rendering procedures in inactivating bovine spongiform encephalopathy (BSE) and scrapie agents. Vet. Rec. 142 : 474-480.
- Scientific Opinion of the Panel on Biological Hazards (2007) Certain aspects related to the feeding of animal proteins to farm animais. EFSA J. 576 : 1-41.
- Taguchi, F., Tamai, Y., Uchide, K., Kitajima, R., Kojima, H., Kawaguchi, T., Ohtani, Y. and Miura, S. (1991) Proposai for a procédure for complete inactivation of the Creutzfeldt-Jakob disease agent. Arch. Virol. 119 : 297-301.
- Taylor, D.M. and McConnell, I. (1988) Autoclaving does not decontaminate formol-fixed scrapie tissues. The Lancet 1(8600): 1463-1464.
- Taylor, D.M., Fraser, H., McConnell, I., et al. (1994) Decontamination studies with the agents of bovine spongiform encephalopathies and scrapie. Arch Virol 139: 313-326.
- Taylor, D.M., Woodgate, S.L. and Atkinson, M.J. (1995) inactivation of the bovine spongiform encephalopathy agent by rendering procedures. Vet. Rec. 137: 605-610.
- Taylor, D.M., McConnell, I and Fernie, K. (1996) The effet of dry heat on the ME7 strain of mouse-passaged scrapie agent. J. Gen. Virol. 77 : 3161-3164.
- Taylor, D.M., Fernie, K. and mcConnell, I. (1997a) Inactivation of the 22A strain of scrapie agent by autoclaving in sodium hydroxide. Vet. Microbiol. 58 : 87-91.
- Taylor, D.M., Woodgate, S.L., Fleetwood, A.J. and Cawthorne, R.J.G. (1997) Effect of rendering procedures on the scrapie agent. Vet. Rec. 141: 643-649.
- Taylor, D.M., Fernie, K., McConnell, I., Ferguson, C.E. and Steele, P.J. (1998) Solvent extraction as an adjunct to rendering : the effect on BSE and scrapie agents of hot solvents followed by dry heat and steam. Vet. Rec. 143: 6-9.
- Taylor, D.M. and Woodgate, S.L. (2003) Rendering practices and inactivation of transmissible spongiform encephalopathy agents. Rev. Sci. Tech. Off. Int. Epiz. 22(1) : 297-310.
- Vadrot, C. and Darbord, J.C. (2006) Quantitative evaluation of prion inactivation comparing steam sterilization and chemical sterilants: proposed method for test standardization. J. Hosp. Infect. 64: 143-148.
- Vilotte, J.L., Soulier, S., Essalmani, R., Stinnakre, M.G., Vaiman, D., Lepourry, L., Costa da Silva, J., Besnard, N., Dawson, M., Buschmann, A., Groshup, M., Petit, S., Madelaine, M.F., Rakatobe, S., Le Dur, A., Vilette, D. and Laude, H. (2001) Markedly increased susceptibility to natural sheep scrapie of transgenic mice expressing ovine PrP. J. Virol. 75 : 5977-5984.
- WHO Infection Control Guidelines for transmissible Spongiform Encephalopathies. Report of a WHO consultation, Geneva, Switzerland, 23-26 March 1999. WHO/CDS/CSR/APH/2000.3
- Yan, Z.X., Stitz, L., Heeg, P., Pfaff, E. and Roth, K. (2004) Infectivity of prion protein bound to stainless steel wires : a model for testing decontamination procedures for transmissible spongiform encephalopathies. Infect. Control Hosp. Epidemiol. 25 : 280-283.
- Zobeley, E., Flechsig, E., Cozzio, A., Enari, M.and Weissmann, C. (1999) Infectivity of scrapie prions bound to a stainless steel surface. Mol. Med. 5 : 240-243.

## Revendications

1. Procédé de décontamination d'une surface susceptible d'être contaminée par un agent pathogène comprenant :
- dans une première étape, la mise en contact de la surface avec une première solution aqueuse ayant un pH supérieur ou égal à 9,5 et inférieur à 13, comprenant au moins une base faible ayant au moins un pKa compris entre 8 et 13 et dont la concentration dans la solution est d'au moins 5.10⁻³mol.l⁻¹, la première solution ne contenant pas d'agent oxydant ;
- suivie, d'une deuxième étape comprenant la mise en contact de la surface avec une deuxième solution aqueuse comprenant un agent oxydant à une concentration d'au moins 50 ppm.

2. Procédé selon la revendication 1, dans lequel le pH de la première solution est inférieur ou égal à 12,5.

3. Procédé selon la revendication 1 ou 2, dans lequel le pH de la première solution est supérieur ou égal à 10,5.

4. Procédé selon l'une des revendications 1 à 3, dans lequel la durée de la première étape est d'au moins 5 minutes, de préférence au moins 15 minutes.

5. Procédé selon l'une des revendications 1 à 4, dans lequel la durée de la deuxième étape est d'au moins 5 minutes.

6. Procédé selon l'une des revendications 1 à 5, dans lequel le procédé est mis en œuvre à une température de 18 °C à 25 °C.

7. Procédé selon l'une des revendications 1 à 6, dans lequel la température de la première solution et/ou de la deuxième solution au moment de la mise en contact avec la surface est d'au moins 40°C.

8. Procédé selon l'une des revendications 1 à 7, dans lequel la première étape comprend également la mise en contact de la surface avec un détergent.

9. Procédé selon la revendication 8, dans lequel le détergent est sélectionné dans le groupe constitué d'un octylphénol éthoxylé, d'un alkylsulfate de sodium, et de dodécyl sulfate de sodium.

10. Procédé selon l'une des revendications 1 à 9, dans lequel la première étape comprend également la mise en contact de la surface avec un agent chélatant.

11. Procédé selon la revendication 10, dans lequel l'agent chélatant est sélectionné dans le groupe constitué de 1-hydroxy éthylidène-1,1-diphosphonate (HEDP), de méthylglycinediacétate et d'éthylène diamine tétra-acétate (EDTA).

12. Procédé selon l'une des revendications 1 à 11, dans lequel la base faible est un sel de carbonate.

13. Procédé selon l'une des revendications 1 à 12, dans lequel l'agent oxydant est sélectionné dans le groupe constitué d'un sel d'hypochlorite, d'un sel de dichloro isocyanurate, du peroxyde d'hydrogène, et d'un peracide.

14. Procédé selon l'une des revendications 1 à 13, dans lequel l'agent pathogène est sélectionné dans le groupe constitué d'un prion, d'un virus, d'une bactérie, d'un champignon, d'un protozoaire, et d'une spore de champignon ou de bactérie.

15. Procédé selon la revendication 14, dans lequel l'agent pathogène est un prion.

## Patentansprüche

1. Verfahren zur Dekontaminierung einer möglicherweise mit einem pathogenen Erreger kontaminierten Oberfläche, das Folgendes umfasst:
- in einer ersten Phase das Inkontaktbringen der Oberfläche mit einer ersten wässrigen Lösung mit einem pH-Wert höher oder gleich 9,5 und geringer als 13, die zumindest eine schwache Base, die zumindest einen pKs-Wert zwischen 8 und 13 aufweist und deren Konzentration in der Lösung bei zumindest 5.10⁻³mol.l⁻¹ liegt, umfasst, wobei die erste Lösung kein Oxidationsmittel enthält;
- gefolgt von einer zweiten Phase, die das Inkontaktbringen der Oberfläche mit einer zweiten wässrigen Lösung, die ein Oxidationsmittel mit einer Konzentration von zumindest 50 ppm umfasst, umfasst.

2. Verfahren nach Anspruch 1, worin der pH-Wert der ersten Lösung geringer oder gleich 12,5 ist.

3. Verfahren nach Anspruch 1 oder 2, worin der pH-Wert der ersten Lösung größer oder gleich 10,5 ist.

4. Verfahren nach einem der Ansprüche 1 bis 3, worin die Dauer der ersten Phase zumindest 5 Minuten, bevorzugt zumindest 15 Minuten, beträgt.

5. Verfahren nach einem der Ansprüche 1 bis 4, worin die Dauer der zweiten Phase zumindest 5 Minuten beträgt.

6. Verfahren nach einem der Ansprüche 1 bis 5, worin das Verfahren bei einer Temperatur von 18 °C bis 25 °C umgesetzt wird.

7. Verfahren nach einem der Ansprüche 1 bis 6, worin die Temperatur der ersten Lösung und/oder der zweiten Lösung zum Zeitpunkt des Inkontaktbringens mit der Oberfläche zumindest 40 °C beträgt.

8. Verfahren nach einem der Ansprüche 1 bis 7, worin die erste Phase auch das Inkontaktbringen der Oberfläche mit einem Detergens umfasst.

9. Verfahren nach Anspruch 8, worin das Detergens in der Gruppe ausgewählt wird, die aus einem ethoxylierten Octylphenol, einem Natriumalkylsulfat und einem Natriumdodecylsulfat besteht.

10. Verfahren nach einem der Ansprüche 1 bis 9, worin die erste Phase auch das Inkontaktbringen der Oberfläche mit einem chelatbildenden Mittel umfasst.

11. Verfahren nach Anspruch 10, worin das chelatbildende Mittel in der Gruppe ausgewählt wird, die aus 1-Hydroxyethan-1,1-diphosphonsäure (HEDP), Methylglycindiacetat und Ethylendiamintetraacetat (EDTA) besteht.

12. Verfahren nach einem der Ansprüche 1 bis 11, worin die besagte schwache Base ein Carbonatsalz ist.

13. Verfahren nach einem der Ansprüche 1 bis 12, worin das Oxidationsmittel in der Gruppe ausgewählt wird, die aus einem Hypochloritsalz, einem Dichlorisocyanuratsalz, Wasserstoffperoxid und einer Persäure besteht.

14. Verfahren nach einem der Ansprüche 1 bis 13, worin der pathogene Erreger in der Gruppe ausgewählt wird, die aus einem Prion, einem Virus, einer Bakterie, einem Pilz, einem Protozoon und einer Pilz- oder Baktierenspore besteht.

15. Verfahren nach Anspruch 14, worin es sich bei dem pathogenen Erreger um ein Prion handelt.

## Claims

1. A method of decontaminating a surface liable to be contaminated by a pathogenic agent comprising:
- in a first step, bringing the surface into contact with a first aqueous solution having a pH greater than or equal to 9.5 and less than 13, comprising at least one weak base having at least one pKa of between 8 and 13 and of which the concentration in the solution is at least 5.10⁻³ mol.l⁻¹, the first solution not containing an oxidising agent;
- followed by a second step comprising bringing into contact the surface with a second aqueous solution comprising an oxidising agent at a concentration of at least 50 ppm.

2. The method according to claim 1, wherein the pH of the first solution is 12.5 or less.

3. The method of claim 1 or 2, wherein the pH of the first solution is greater than or equal to 10.5.

4. The method according to one of claims 1-3, wherein the duration of the first step is at least 5 minutes, preferably at least 15 minutes.

5. The method according to one of claims 1-4, wherein the duration of the second step is at least 5 minutes.

6. The method according to one of claims 1-5, wherein the method is carried out at a temperature of 18°C to 25°C.

7. The method according to one of claims 1-6, wherein the temperature of the first solution and/or the second solution at the time of bringing into contact the surface is at least 40°C.

8. The method according to one of claims 1-7, wherein the first step also comprises bringing into contact the surface with a detergent.

9. The method according to claim 8, wherein the detergent is selected from the group consisting of an ethoxylated octylphenol, a sodium alkyl sulphate, and sodium dodecyl sulphate.

10. The method according to one of claims 1-9, wherein the first step also comprises bringing into contact the surface with a chelating agent.

11. The method according to claim 10, wherein the chelating agent is selected from the group consisting of 1-hydroxy ethylidone-1,1-diphosphonate (HEDP), methylglycinediacetate and ethylenediaminetetraacetate (EDTA).

12. The method according to one of claims 1-11, wherein the weak base is a carbonate salt.

13. The method according to one of claims 1-12, wherein the oxidising agent is selected from the group consisting of a hypochlorite salt, a dichloro isocyanurate salt, hydrogen peroxide, and a peracid.

14. The method according to one of claims 1-13, wherein the pathogenic agent is selected from the group consisting of a prion, a virus, a bacterium, a fungus, a protozoan, and a fungal or bacterial spore.

15. The method according to claim 14, wherein the pathogenic agent is a prion.
